(19) Europäisches Patentamt
European Patent Office
Office européen des brevets

(11) Publication number: **0 021 497**
**A1**

# (12) EUROPEAN PATENT APPLICATION

(21) Application number: 80200533.0

(22) Date of filing: 10.06.80

(51) Int. Cl.³: C 07 C 43/13
C 07 C 41/16, C 08 G 65/32

(30) Priority: 21.06.79 US 50840

(43) Date of publication of application: 07.01.81 Bulletin 81/1

(84) Designated Contracting States: BE DE FR GB IT NL

(71) Applicant: THE PROCTER & GAMBLE COMPANY
301 East Sixth Street
Cincinnati Ohio 45202(US)

(72) Inventor: Gibson, Thomas Wiliam
2659 W. Kemper Road
Cincinnati Ohio 45231(US)

(74) Representative: Gibson, Tony Nicholas et al,
Procter & Gamble (NTC) Limited Whitley Road
Longbenton Newcastle upon Tyne NE12 9TS(GB)

## (54) Synthesis of polyoxyalkylene glycol monoalkyl ethers.

(57) A solvent-free method for preparing monoalkyl ethers of polyoxyalkylene glycols, comprising mixing a polyoxyalkylene glycol, especially polyoxyethylene glycol, with a concentrated aqueous solution of a metal hydroxide, contacting said mixture with a primary alkyl halide, and recovering the monoalkyl ether.

EP 0 021 497 A1

# SYNTHESIS OF POLYOXYALKYLENE GLYCOL MONOALKYL ETHERS

## Technical Field

Commercial processes for producing monoalkyl ethers of polyoxyalkylene glycols condense the corresponding alkylene oxide with a primary alcohol to produce compounds having varying oxyalkylene chain lengths. For example, the condensation of lauryl alcohol with ethylene oxide will yield a lauryl ethoxylate having a Poisson distribution of chain lengths.

For some applications, it is desirable and necessary to have monoalkyl ethers of polyoxyalkylene glycols with a homogeneous oxyalkylene chain length. U.S. Patent 4,067,961, Laughlin, January 10, 1978, discloses the use of monodecyl tetraoxyethylene glycol and monodecyl pentaoxyethylene glycol as spermicides in contraceptive devices. British Patent 1,462,134, Collins, January 19, 1977, discloses the superiority of a narrowly defined range of monoalkyl ethers, especially monodecyl tetraoxyethylene glycol, in cleaning organic hydrophobic soils from synthetic fabrics.

Homogeneous monoalkyl ethers have been produced by two general types of reactions, by the reaction of an alkyl paratoluenesulfonate with a polyalkylene glycol, or by a Williamson ether synthesis reaction using an alkyl bromide and an alkali metal polyoxyalkylene glycoxide.

In Williamson ether syntheses, the alkoxylate is usually formed by the reaction of glycol with alkali metal or alkali metal hydride to generate the glycoxide, followed by reaction with an alkyl halide, all in a nonaqueous medium. Traditional Williamson ether synthesis

reactions have the disadvantages of requiring the handling of large amounts of sodium or sodium hydrides and producing products contaminated with lower alkoxylogs and with dialkylated products.

Recently, Williamson ether syntheses have been performed using phase transfer techniques. A heterogeneous reaction mixture, usually having an organic solvent phase and a concentrated aqueous hydroxide phase, together with a phase transfer catalyst has been used to prepare ethers under mild conditions.

Although aqueous conditions can be used and reaction conditions are relatively mild, phase transfer catalysis conditions have the disadvantage of generally requiring the use of a solvent, e.g. tetrahydrofuran, benzene, methylene chloride, ethyl acetate, dimethyl sulfoxide, as well as the use of a catalyst which must ultimately be separated from the product. Typical phase transfer catalysts are tetrasubstituted ammonium halides and sulfates ($Q^+$), e.g. tetrabutylammonium bromide or bisulfate, or benzyltriethylammonium chloride. Crown ethers and cryptands have also been used as phase transfer catalysts. It has been suggested in the literature that phase transfer monoalkylation reactions involving diacids could be performed by strict control of the pH of the reaction mixture or by chemical reaction of the catalyst to "poison" it. However, the acidity of the polyoxyalkylene glycols is too low to take advantage of the pH effect. Additionally, a full equivalent of $Q^+$ would be required.

The present invention reflects the discovery that under Williamson phase transfer/extractive alkylation conditions, a polyoxyalkylene glycol performs not only as a reactant, but also as its own phase transfer catalyst. Through the present invention, monoalkyl ethers of polyoxyalkylene glycols having homogeneous chain lengths are conveniently produced in high yields and excellent

purity under phase transfer/extractive alkylation conditions using purely stoichiometric control of the reactants. Additionally, the reaction proceeds without using extraneous organic solvents or extraneous phase transfer catalysts so that isolation and purification procedures can be greatly simplified.

## Background Art

Recent Williamson ether syntheses have been performed using phase transfer techniques with heterogeneous reaction mixtures, organic solvent and concentrated aqueous hydroxide, and a "phase transfer catalyst." Freedman, et al., Tet. Ltr., 3251 (1975).

The phase transfer catalyst forms an ion pair (with the water-soluble/organically insoluble alkoxide) which preferentially partitions into the organic layer where reaction occurs. The strict exclusion of water required by the traditional Williamson procedure is not necessary and reaction conditions are relatively mild. The most widely used phase transfer catalysts are tetraalkylammonium salts, although crown ethers, cryptands, open chain polyethers (Yanagida, et al., Bull. Chem. Soc. Jap., 50, 1386 (1977)), polyether substituted triazines and pentaerythritols (Fornasier, et al., Tet. Ltr., 1381 (1976)), "octopus" polyether molecules (Vogtle, et al., Angew. Chem. Int. Ed., 13, 814 (1974)) and dialkyl polyethylene glycols (Lee, et al., J. Org. Chem., 43, 1532 (1978), Yanagida, Tet. Ltr., 2893 (1977) and Lehmkuhl, et al., Synthesis, 184 (1977)) have also been used as catalysts.

Brandstrom, in Adv. in Phys. Org. Chem., 15, 267 (1977), suggests the use of extractive alkylation conditions (a 1:1 mole ratio of catalyst:reactant) instead of phase transfer catalytic conditions (a 1:20 mole ratio of catalyst:reactant) to achieve selective alkylation of diacids. However, production of pure monoalkyl compound is achieved either by chemical reaction of catalyst, present in stoichiometric (as opposed to catalytic) amounts, with halide to "poison" it, or by strict pH control.

U.S. 4,061,682, Dubois, et al., Dec. 12, 1977, discloses the preparation of symmetrical ethers using

alkyl or aryl halides, sulfates or sulfonates in the presence of an aqueous alkali or alkaline earth hydroxide, carboxylate salt and a quaternary ammonium or phosphonium catalyst. U.S. 3,914,320, Gordon, Oct. 21, 1975, also relates to the preparation of symmetrical ethers using halogenated hydrocarbons, alkali metal hydroxide and a quaternary salt catalyst.

Japanese Patent J5 3133-300 (Derwent 00982B/01) Kuraray KK, published November 20, 1978, discloses the preparation of dialkyl ethers of polyoxyalkylene compounds using an organohalide, aqueous NaOH or KOH, and a reducing agent. The use of the reducing agent is disclosed as being the improvement in the process of Japanese application 86999/76, now Japanese Patent J5 3011-997 (Derwent 20597A/11) Kuraray KK, published February 2, 1978.

Several older patents teach the preparation of monoalkylethoxylates through reaction of alkali metal hydroxide, alkyl or aralkyl halide, and polyol; the reaction conditions are non-aqueous. U.S. 2,596,091, de Benneville, May 13, 1952, and U.S. 2,596,093, de Benneville, May 13, 1952.

None of the workers in the art appear to have appreciated the benefits and requirements of Applicant's solvent-free etherification process.

## Disclosure of the Invention

The present invention encompasses a solvent-free method for preparing monoalkyl ethers of polyoxyalkylene glycols selected from the group consisting of polyoxyethylene glycols, polyoxypropylene glycols and mixed polyoxyethylene-polyoxypropylene glycols comprising mixing the polyoxyalkylene glycol with a concentrated aqueous solution of a metal hydroxide, contacting said mixture with an organohalide in a mole ratio of glycol to halide greater than about 2:1 and a mole ratio of hydroxide to halide greater than about 1:1, and recovering the monoalkyl polyoxyalkylene glycol ether. The outstanding features of this invention are that monoethers of glycols can be easily prepared through purely stoichiometric control of the reactants and that extraneous solvents and catalysts are not needed for the process. Additionally, typical phase transfer catalysts and solvents can greatly decrease the yield of final product.

The process of the present invention can be illustrated by the reaction of decyl bromide with tetraoxyethylene glycol to obtain monodecyl tetraoxyethylene glycol:

$$CH_3(CH_2)_8CH_2Br + HO(CH_2CHO)_4H \xrightarrow[OH^-]{} CH_3(CH_2)_8CH_2O(CH_2CHO)_4H.$$

By "polyoxyalkylene glycol" as used herein is meant a compound of the general formula $HO(C_nH_{2n}O)_xH$ wherein n is 2 or 3. The glycol can be of uniform n, for example polyoxyethylene glycol (n=2) and polyoxypropylene glycol (n=3), or the glycol can have an n of mixed integers, i.e. a mixture of oxyalkylene components, for example $HO(C_2H_4O)_a-(C_3H_6O)_b-(C_2H_4O)_a-H$. The polyoxyalkylene glycols used herein are polyoxyethylene glycols, polyoxypropylene glycols and mixed polyoxyethylene/polyoxypropylene glycols. The most preferred glycols are

polyoxyethylene glycols. The number of oxyalkylene units in the glycol (x) can be 2 or greater, e.g. monoalkyl ethers of dioxyethylene glycol and dodecaoxyethylene glycol can be easily obtained.

The metal hydroxide used herein is typically an alkali metal hydroxide or an alkaline earth metal hydroxide, magnesium hydroxide, lithium hydroxide, barium hydroxide, for example. The preferred metal hydroxides are alkali metal hydroxides and the most preferred hydroxides are sodium hydroxide and potassium hydroxide.

The organohalides used herein can be any convenient organic halide that does not undergo base catalyzed reactions. Straight chain alkyl halides of chain length $C_2$ and greater which have a melting point below the temperature at which the reaction of the glycol/hydroxide mixture with the halide is to be performed are the preferred organohalides. Typical halides include ethyl chloride, dodecyl bromide, and stearyl iodide. The preferred alkyl halides are of chain length $C_5$-$C_{15}$ and the most preferred are of chain length $C_8$-$C_{14}$.

The present invention can also be used to extend polyoxyalkylene glycol chain lengths by reaction of shorter-chain polyoxyalkylene glycols with a terminal dihalopolyoxyalkylene ether. For example, a polyoxyethylene glycol of the above general formula having 9 oxyalkylene units (x=9) can be prepared from two glycols having 3 oxyalkylene units (x=3) through reaction with $Cl(C_2H_4O)_2CH_2CH_2Cl$. The terminal dihalopolyoxyalkylene ether is of the general formula $Y(C_nH_{2n}O)_xC_2H_{2n}Y$ wherein Y is halogen. As with the polyoxyalkylene glycols, the polyoxyalkylene ether portion of the terminal dihalopolyoxyalkylene ether can also have uniform or mixed n, oxyalkylene groups. The preferred dihalo ethers have n=2 or 3 or mixtures, i.e. polyoxyethylene, polyoxypropylene, or mixed polyoxyethylene/propylene. The preferred halides are the chloride and the bromide and the preferred dihalo ethers are dihalopolyoxyethylene ethers.

By "comprising" as used herein is meant that other steps and materials can be used and present so long as they do not adversely affect the desirable characteristics of the invention. Thus the term "comprising" encompasses the more restrictive terms "consisting essentially of" and "consisting of."

The following general procedure is applicable to the preparation of any monoalkyl ether of a polyoxyalkylene glycol. A mixture of concentrated aqueous metal hydroxide and glycol is heated for about one-half hour in an oil bath at the temperature at which the reaction is to be performed. The mixture is stirred while a stream of argon flushes air from the system. Alkyl halide is added and the reaction is allowed to proceed for the desired time. After cooling, the mixture is diluted with water, extracted exhaustively with any suitable organic solvent, e.g. ether, toluene or benzene, dried over a suitable drying agent, e.g. magnesium sulfate, filtered and stripped on a rotary evaporator.

The metal hydroxide is typically used in concentrated aqueous solution, preferably greater than about 30% by weight and most preferably 50% or greater by weight. The greater the amount of water present in the reaction mixture, the slower the rate of reaction and the more side products formed.

The metal hydroxide is used at a mole ratio of hydroxide:alkyl halide of greater than about 1:1. The yield of pure monoalkyl alkyoxylate is substantially unaffected with any more than about a 5% molar excess of hydroxide as compared to alkyl halide (1.05:1). Since the rate of the reaction will increase with increasing molar amounts of hydroxide, a mole ratio of hydroxide: halide of greater than about 5:1 increases the rate of reaction significantly. However, because it is easier to remove and recover unreacted glycol in subsequent purification procedures where less base is used, the preferred hydroxide:halide mole ratio is from about 1.05:1 to about 2:1.

The mixing of glycol and hydroxide can take place in any convenient manner that will effect an intimate mixture of the components. Typically, mechanical agitation by stirring is adequate. After the hydroxide and glycol are mixed, air is flushed from the system by inert gas sparging; argon is convenient. Unless air is removed from the reaction vessel, there is a tendency to form undesirable side products. The agitation of the inert gas flush also provides some mixing of the glycol and hydroxide.

After the reaction vessel and mixture are sparged, the organohalide, preferably an alkyl halide, is added. The alkyl halide is most preferably primary since secondary alkyl halides are subject to elimination reactions giving undesirable side products. The most preferred alkyl halides are alkyl bromides and chlorides. Alkyl compounds having "soft" leaving groups such as iodides can be used in the reactions of the present invention but they tend to have undesirable hydrolysis and elimination reactions which give side products. Generally the alkyl chlorides and the alkyl bromides are equally desirable, but at mole ratios of glycol:alkyl halide of from about 2:1 to about 5:1, alkyl chlorides are preferred since alkyl bromides have a greater tendency to form dialkyl ethers instead of pure monoalkyl ethers. The mole ratio of glycol:alkyl halide is greater than about 2:1, preferably greater than about 5:1 and is most preferably greater than about 10:1.

The alkyl halide is added to the concentrated aqueous hydroxide/glycol mixture in the reaction vessel. It is to be understood that the reaction mixture is a two-phase system after the addition of the halide. The reaction of halide with glycol proceeds as the glycol functions as its own phase transfer catalyst by which an

ion pair of glycol and glycoxide passes into the halide phase for reaction. Mechanical agitation is generally desirable to promote thorough mixing of the reactants, but such agitation does not affect the rate of the phase transfer reaction.

Where the mole ratio of glycol:alkyl halide is greater than about 10:1 and the ratio of hydroxide:alkyl halide is greater than about about 1.05:1, there are substantially no side reactions or production of dialkyl ethers.

The reaction of alkyl halide and glycol is convenient to carry out, requiring only the mixing of glycol and sodium hydroxide solution with the halide, followed by heating for, routinely, 8 to 24 hours at a convenient temperature, usually in the range of 60°C to 120°C, but typically 100°C.

The product can be recovered and purified using standard synthetic techniques. Typical recovery and purification procedures include solvent partition for extraction of the monoalkyl ether, drying, filtration, solvent stripping and further purification techniques.

Where longer chain polyoxyalkylene glycols are being prepared from shorter chain polyoxyalkylene glycols and terminal dihalopolyoxyalkylene glycol ethers, the same procedures are followed as in synthesizing the monoalkyl ethers.

Additionally, monoalkyl ethers of ethylene glycol can be prepared using the same procedures as are used in preparing monoalkyl ethers of polyoxyalkylene glycols.

## Best Mode

### Preparation of Monodecyltetraethylene Glycol

A mixture of 194 g. (1.0 mole) of tetraethylene glycol and 8.4 g. (0.105 mole) of 50% aqueous NaOH was heated to 100°C under argon. Decyl bromide (22.1 g., 0.10 mole) was added and the mixture (mole ratio glycol:hydroxide:halide = 10:1.05:1) was stirred at 100°C for 24 hours. The cooled mixture was extracted 4 times with ether, the combined extracts were dried over $MgSO_4$, filtered, and the solvent removed in vacuum. The residue was dissolved in 240 ml. of 3:1 v/v $MeOH:H_2O$ and extracted twice with pentane. The aqueous methanol solution was stripped and distilled to give 29.2 g. (88%) of the title monoether, b.p. 145-152°C (50 μ). Gas chromatographic analysis revealed a purity of greater than 98%.

Table I illustrates the effects of using decyl chloride in place of decyl bromide following the procedure above, as well as the effect of varying the mole ratio of glycol to halide.

Table I

Effects of Varying Halide and of Glycol:Halide Mole Ratio (Mole Ratio NaOH:Halide = 1)

| Alkyl Halide | Glycol | Mole Ratio Glycol: Halide | % Mono-alkylation | % Di-alkylation |
|---|---|---|---|---|
| Decyl chloride | Diethylene | 2 | 44 | 20 |
| " " | " | 10 | 80 | - |
| " " | Tetraethylene | 2 | 59 | 26 |
| " " | " | 10 | 85 | - |
| Decyl bromide | Diethylene | 2 | 21 | 63 |
| " " | " | 10 | 79 | - |
| " " | Tetraethylene | 2 | 42 | 48 |
| " " | " | 3 | 64 | 26 |
| " " | " | 10 | 88 | - |

## Industrial Applicability

### Example I

### Preparation of Monobutyldiethylene Glycol

A mixture of 106 g. (1.0 mole) of diethylene glycol and 8.3 g. (0.104 mole) of 50% aqueous NaOH was heated to 100°C with stirring under an argon atmosphere. Butyl chloride (9.2 g., 0.10 mole) was added and the mixture was maintained at 100°C for 24 hours. (Mole ratio glycol:hydroxide:halide = 10:1.04:1.) The cooled mixture was extracted 4 times with ether, the combined extracts dried over $MgSO_4$, and distilled to give 13.7 g. of monoether, b.p. 70-80°C (0.9 mm.). Gas chromatographic analysis of the trimethylsilyl ethers showed the material to be 89% pure, the only contaminant being diethylene glycol, for a yield of 75%.

### Example II

### Preparation of Monodocosyltetraethylene Glycol

The crude product from reaction of 0.05 mole of docosyl bromide, 0.05 mole of 50% NaOH, and 0.10 mole of tetraethylene glycol at 100°C for 24 hours, following the procedure of Example I, was dissolved in methylene chloride. (Mole ratio glycol:hydroxide:halide = 2:1:1.) Chromatography of an aliquot of this solution on silica gel gave docosyl bromide and docosene in 10% yield, docosylether in 10% yield, didocosyltetraethylene glycol, m.p. 69-70°C (39%), and monodocosyltetraethylene glycol, m.p. 59-60°C (38%).

### Example III

### Preparation of Nonaethylene Glycol

A mixture of triethylene glycol (300 g., 2.0 moles), 50% aqueous NaOH (17.0 g., 0.21 mole), and ethylene glycol-bis-(2-chloroethyl)ether (18.6 g., 0.10 mole) was heated at 100°C for 24 hours. (Mole ratio glycol:hydroxide:halide = 10:1.05:0.9.) The mixture was cooled to

room temperature, neutralized to pH 7 with 1N HCl, and dissolved in tetrahydrofuran to precipitate salts. The residue obtained after filtration and solvent removal was dissolved in 400 ml. $H_2O$ and treated with Rexyn 300 mixed-bed ion exchange resin until the conductivity dropped to 0.3 micromho/cm. Filtration and distillation gave 264.6 g. of triethylene glycol and 35.3 g. (85%) of nonaethylene glycol. The product was contaminated with small amounts of penta-, hexa- and octaethylene glycols, which could be removed by crystallization from 1:1 ether:tetrahydrofuran at 0°C.

The following tables reflect experiments using butyl chloride and diethylene glycol. Table II illustrates the effect of glycol:halide:hydroxide ratios on yield of monoalkyl ether as well as the adverse effect of extraneous solvent, which in these experiments was benzene, a typical phase transfer solvent.

Table II

Effect of Glycol:Halide Ratio on Alkylation of Diethylene Glycol with Butylchloride*

| Exp. | Mole Ratio Glycol: Halide | Solvent | % Dialkylation | % Monoalkylation |
|---|---|---|---|---|
| 1 | 0.25 | None | 97 | - |
| 2 | 1.0 | " | 70 | 9 |
| 3 | 5.0 | " | 13 | 38 |
| 4 | 10.0 | " | - | 40 |
| 5 | 1.0 | Benzene | 10 | 6 |
| 6 | 5.0 | " | 2 | 8 |
| 7 | 10.0 | " | - | 8 |

*60°, 4 hrs.,; mole ratio NaOH:Halide = 5, tetrabutylammonium bisulfate:halide = 0.1.

Table III illustrates the adverse effects of typical phase transfer solvents and a typical $Q^+$ phase

transfer catalyst on the reaction of butyl chloride and diethylene glycol.

Table III

Effect of Solvent and Phase Transfer Catalyst on Yield of Monoalkylation Product*

| Solvent | Catalyst | $Bu(OCH_2CH_2)_2OH$ Yield |
|---|---|---|
| None | None | 69 |
| " | TBAB** | 63 |
| Benzene | None | 17 |
| " | TBAB** | 26 |
| THF*** | None | 30 |
| " | TBAB** | 47 |

* 60°, 16 hrs., mole ratio BuCl:glycol:NaOH = 1:10:5

** Tetrabutylammoniumbisulfate (0.1:1 mole ratio TBAB:BuCl)

***Tetrahydrofuran

The etherification reaction can be performed using a wide variety of organohalides, for example substituted or unsubstituted alkyl, alkenyl, aryl, aralkyl, cycloalkyl. The only requirements are that the organohalide have a melting point below the temperature at which the reaction is to take place and that it not have substituents which are subject to base catalyzed reactions.

CLAIMS

1. A solvent-free method for preparing monoalkyl ethers of polyoxyalkylene glycols, comprising:

(a) mixing a polyoxyalkylene glycol selected from the group consisting of polyoxyethylene glycols, polyoxypropylene glycols, and mixed polyoxyethylene-polyoxypropylene glycols with a concentrated aqueous solution of a metal hydroxide; and

(b) contacting the mixture of Step (a) with an organohalide in a mole ratio of glycol to halide greater than 2:1 and a mole ratio of hydroxide to halide greater than about 1:1.

2. A method according to Claim 1 wherein the metal hydroxide is an alkali metal hydroxide or an alkaline earth metal hydroxide.

3. A method according to Claim 2 wherein the metal hydroxide is selected from sodium hydroxide and potassium hydroxide.

4. A method according to any one of Claims 1-3 wherein said organohalide is a primary alkyl halide.

5. A method according to Claim 4 wherein the alkyl halide is an alkyl chloride or bromide.

6. A method according to any one of Claims 1-5 wherein the mole ratio of glycol to halide is greater than 5:1.

7. A method according to any one of Claims 1-6 wherein the mole ratio of hydroxide to halide is from 1.05:1 to 3:1.

8. A method according to any one of Claims 1-6 wherein the mole ratio of hydroxide to alkyl halide is greater than about 5:1.

9. A method according to any one of Claims 1-3 characterised in that the organo halide is a terminal dihalopolyoxy alkylene ether.

10. A method according to Claim 9 wherein the halogens of said terminal dihalopolyoxyalkylene ether are chlorine or bromine.

European Patent Office

# EUROPEAN SEARCH REPORT

Application number
EP 80 20 0533

## DOCUMENTS CONSIDERED TO BE RELEVANT

| Category | Citation of document with indication, where appropriate, of relevant passages | Relevant to claim |
| --- | --- | --- |
| | No relevant documents have been disclosed. | |

**CLASSIFICATION OF THE APPLICATION (Int. Cl. [3])**

C 07 C 43/13
41/16
C 08 G 65/32

**TECHNICAL FIELDS SEARCHED (Int.Cl. [3])**

C 07 C 43/13
41/16
C 08 G 65/32

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
| --- | --- | --- |
| The Hague | 18-09-1980 | SAGATYS |